# EUROPEAN PATENT SPECIFICATION

(11) **EP 2 505 513 B1**
(45) Date of publication and mention of the grant of the patent: **15.06.2016**
(21) Application number: 10833335.2
(22) Date of filing: 26.11.2010
(51) Int. Cl.: B65D 81/24, A61F 13/15, A61F 13/472, B65D 30/16, B65D 81/26, B65D 75/00, B65D 33/01

(54) **PACKAGE OF ABSORBENT ARTICLE**
VERPACKUNG FÜR EINEN SAUGFÄHIGEN ARTIKEL
CORPS D'EMBALLAGE POUR PRODUIT ABSORBANT

(30) Priority: 27.11.2009 JP 2009270169
(43) Date of publication of application: 03.10.2012
(73) Proprietor: Unicharm Corporation, Ehime 799-0111 (JP)
(72) Inventor: MATSUSHIMA, Azusa, Kanonji-shi Kagawa 769-1602 (JP); YAMAKI, Koichi, Kanonji-shi Kagawa 769-1602 (JP)
(74) Representative: Carr, Anne Marie
(86) International application number: PCT/JP2010/071142
(87) International publication number: WO 2011/065489

(56) References cited:
- EP-A1- 2 128 037
- DE-A1- 10 101 661
- JP-A- 2003 191 963
- JP-A- 2005 335 790
- JP-A- 2005 514 272
- JP-A- 2009 132 403
- JP-U- H0 724 764
- US-A1- 2003 053 723
- US-A1- 2005 288 644

## Description

### [Field of the Invention]

The present invention relates to a package of an absorbent article in which the absorbent article having an absorber is packaged in a sealed state.

### [Background Art]

An absorbent article such as a sanitary napkin is generally sold and stored in the form of a package where an appropriate number of absorbent articles are placed in a packaging bag, for example. Document US 2005/0288644 A1 discloses a package according to the preamble of claim 1.

A structure in which a ruffle (extended support unit) extending out from a bottom surface of the packaging bag is provided is known as an example of such a packaging bag of an absorbent article (see Patent Document 1). According to such a packaging bag, because the packaging bag can be a standalone unit, it comes in handy at the time of selling and storing a package of an absorbent article packaged by the packaging bag.

However, if an absorbent article having an absorber for absorbing the moisture of the bodily fluid, for example, is packaged using the aforementioned conventional packaging bag, below-mentioned problems are encountered. That is, an absorbent article such as a sanitary napkin is generally stored at a location with high humidity and large temperature difference such as a dressing room or a washroom near a bathroom. Because a packaging bag is formed by a resin sheet, for example, and it is difficult to achieve complete water resistance, if a package of an absorbent article is stored at such a location, outside air can enter inside even when the packaging bag has not been opened.

If warm moist air of the bathroom flows into the dressing room having a lower ambient temperature than the bathroom, and comes in contact with a cold wall or floor of the dressing room, or a shelf or window pane inside the dressing room, it builds up condensation. Because the temperature of the placement surface (shelf board surface of the storage shelf or floor) for the packaging bag can be easily transferred to the packaging bag in which the absorbent article is packaged via the space formed between the placement surface and the packaging bag, the air inside the packaging bag that is warmer than the placement surface may build up condensation upon coming in contact with the bottom surface of the packaging bag, when the bottom surface cools to the same temperature as the placement surface.

Thus, if the absorbent article absorbs the moisture formed when the air inside the packaging bag builds up condensation, the absorbent article cannot exhibit the intended absorbency power during use, which is a problem.

### [Related Art Document]

### [Patent Document]

[Patent Document 1] Japanese Patent No. 3965297 (Pages 4 to 6, Fig. 1)

### [Summary of the Invention]

A package (package 1) of an absorbent article according to one characteristic is a package of an absorbent article in which an absorbent article (absorbent article 100) having an absorber (absorber 102) that absorbs moisture is packaged in a sealed state. The package of the absorbent article includes a packaging bag unit (packaging bag unit 10) in which the absorbent article is contained, and an extended support unit (skirt unit 20) extending out from the outer edge portion (outer edge portion E, lateral-side lower edge portions 11c, and bottom-side edge portion 12a) of the bottom surface (bottom surface 12) of the packaging bag unit in a direction that is almost perpendicular to the bottom surface, and configured to support the packaging bag unit to be spaced from a placement surface (placement surface G) on which the package of the absorbent article is placed. The packaging bag unit packages the absorbent article such that the absorber is upright with respect to the bottom surface.

### [Brief Description of the Drawings]

[Fig. 1] Fig. 1 is a perspective view for explaining a package according to an embodiment of the present invention.
[Fig. 2] Fig. 2 (a) is a cross-sectional view in the A - A line of the package according to the embodiment of the present invention, and Fig. 2 (b) is a plan view as seen from the bottom surface of the package according to the embodiment of the present invention (view on arrow B of Fig. 1).
[Fig. 3] Fig. 3 (a) is a perspective view for explaining an example of an absorbent article contained in the package according to the embodiment of the present invention, and Fig. 3 (b) is a cross-sectional view of the absorbent article.
[Fig. 4] Fig. 4 (a) is a schematic diagram for explaining a packaging sheet that enwraps the absorbent article, and Fig. 4 (b) is a perspective view of the absorbent article wrapped in the packaging sheet.
[Fig. 5] Fig. 5 is an enlarged view for explaining a modification of the skirt unit of the package in which a part of the perspective view is enlarged.
[Fig. 6] Fig. 6 is an enlarged view for explaining a modification of the skirt unit of the package in which a part of the perspective view is enlarged.
[Fig. 7] Fig. 7 is an assembly drawing of the package according to the embodiment of the present invention.
[Fig. 8] Fig. 8 is an assembly drawing of the package according to the embodiment of the present invention.
[Fig. 9] Fig. 9 is an assembly drawing of the package according to the embodiment of the present invention.
[Fig. 10] Fig. 10 is an assembly drawing of the package according to the embodiment of the present invention.
[Fig. 11] Fig. 11 is an assembly drawing of the package according to the embodiment of the present invention.
[Fig. 12] Fig. 12 is an assembly drawing of the package for explaining a modification of the method of manufacturing the package.
[Fig. 13] Fig. 13 is a perspective view for explaining a package according to a second embodiment of the present invention.
[Fig. 14] Fig. 14 is an assembly drawing of the package according to the second embodiment of the present invention.
[Fig. 15] Fig. 15 is an assembly drawing of the package according to the second embodiment of the present invention.
[Fig. 16] Fig. 16 is a completion drawing of a continuous body of the package.

### [Embodiments for Carrying out the Invention]

An embodiment of a package according to the present invention is explained with reference to the drawings. In the following description of the drawings, the same or similar reference numerals are used to designate the same or similar parts. It will be appreciated that the drawings are schematically shown and the ratio and the like of each dimension are different from the real ones. Therefore, a specific dimension should be determined in view of the following description. Moreover, among the drawings, the respective dimensional relations or ratios may differ.

### 1. First embodiment

### (Structure of package)

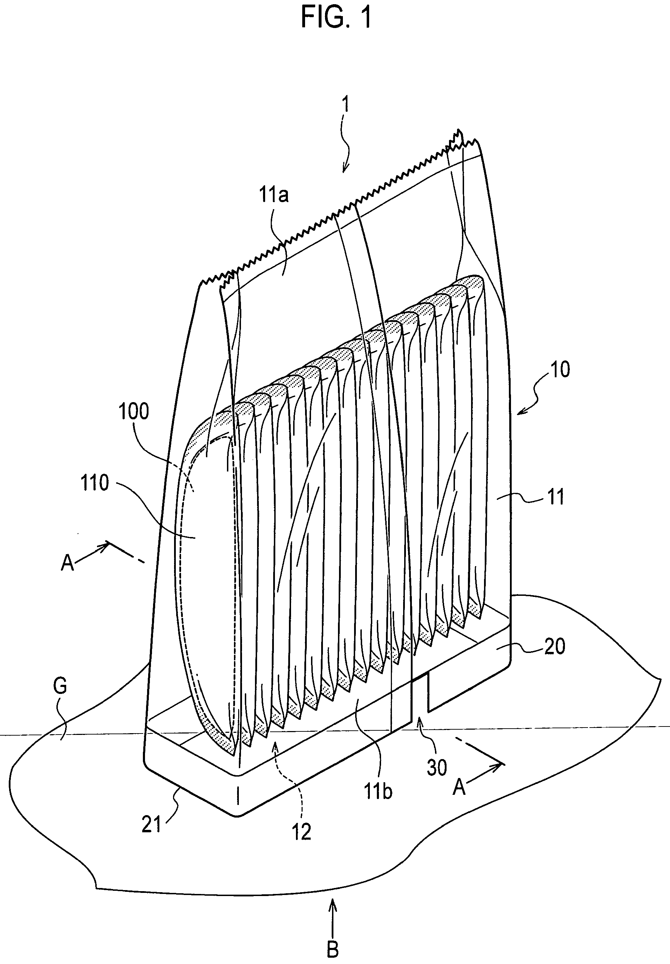

Fig. 1 is a perspective view for explaining a package 1 shown as a first embodiment of the present invention. The package 1 has a packaging bag unit 10 and a skirt unit 20, and packages an absorbent article 100 having an absorber in a sealed state. The absorbent article 100 is contained in the packaging bag unit 20 in an individually packaged state using a packaging sheet 110.

The packaging bag unit 10 is formed in a cylindrical shape from a sheet-like material including a resin sheet. The cylindrical shape includes the round cylindrical shape and the square cylindrical shape. The packaging bag unit 10 includes an end portion 11a and an end portion 11b. The packaging bag unit 10 includes a side surface 11 and a bottom surface 12. The bottom surface 12 is formed by joining one end portion 11b. The side surface 11 of the packaging bag unit 10 has a length such that the absorbent article 100 to be packaged can be contained.

The packaging bag unit 10 packages the absorbent article 100 such that an absorber 102 described later is upright with respect to the bottom surface 12.

An end portion 21 of the skirt unit 20 is in contact with a placement surface G on which the package 1 is placed. The package 1 is supported by the skirt unit 20 and is in an upright state with the bottom surface 12 formed in the end portion 11b of the packaging bag unit 10 at the bottom and the end portion 11a at the top. The bottom surface 12 of the packaging bag unit 10 is spaced from the placement surface G by the skirt unit 20.

In the embodiment, the absorbent article 100 is packaged such that the absorber 102 is in an upright state. The packaging bag unit 10 contains the absorbent article 100 such that the absorbing surface 100c of the absorber 102 is almost perpendicular to the bottom surface 12. In the contained state, the absorbing surface 100c of the absorbent article 100 is almost perpendicular to the bottom surface 12.

Here, the "upright state" includes, as illustrated in the embodiment, the state in which the longitudinal direction of the absorbent article 100 and the up-and-down direction in the state when the package 1 is upright are almost matching. Furthermore, the "upright state" also includes the state when the longitudinal direction of the absorbent article 100 is inclined with respect to the up-and-down direction of the package 1 to an extent where the absorbing surface 100c of the absorbent article does not come in contact with the bottom surface 12.

Furthermore, the "upright state" includes the state in which the widthwise direction perpendicular to the longitudinal direction of the absorbent article 100, or the widthwise direction perpendicular to the longitudinal direction of the absorbent article 100 in a folded state, and the up-and-down direction of the package 1 are almost matching. Furthermore, the "upright state" also includes the state in which the widthwise direction of the absorbent article 100, or the widthwise direction of the absorbent article 100 in a folded state is inclined with respect to the up-and-down direction of the package 1 to an extent where the absorbing surface 100c of the absorbent article does not come in contact with the bottom surface 12.

In the end portion 11a or the side surface 11 near the end portion 11a may be provided with a mechanism (such as a chuck or seal) that enables re-sealing after the seal has been opened once.

The sheet-like material that forms the packaging bag unit 10 is formed by pasting together a single or a plurality of light transmissive or non-light transmissive sheet-like materials. Laminated films described below can be used as the sheet-like material. The films that are laminated are joined with an adhesive with each other.

- Outer layer: OPP (orthophenyl phenol) of thickness 20 µm/adhesive/inner layer: LLDPE (linear low-density polyethylene) of thickness 20 µm
- Outer layer: OPP of 20 µm/adhesive/middle layer: monoaxially-stretched or biaxially-stretched HDPE (high-density polyethylene)/adhesive/inner layer: LLDPE
- Outer layer: OPP of 20 µm/adhesive/middle layer: Al (aluminum) foil/adhesive/inner layer: LLDPE of 20 µm
- Outer layer: OPP in which a silica or alumina deposited layer is formed/adhesive/middle layer: monoaxially-stretched or biaxially-stretched HDPE/adhesive/inner layer: LLDPE of 20 µm
The expression "A/adhesive/B" indicates that A and B are joined with an adhesive. In the above example, a sealant film is used in the inner layer. Thus, the sheet-like material can be joined with a heat seal. Besides the laminated films illustrated here, the sheet-like material can also be a single sealant film. Besides those described above, the laminated films described in Patent Document 1 can be used.

Additionally, a photograph, characters, or a design, for example, may be printed on the surface of the sheet-like material.

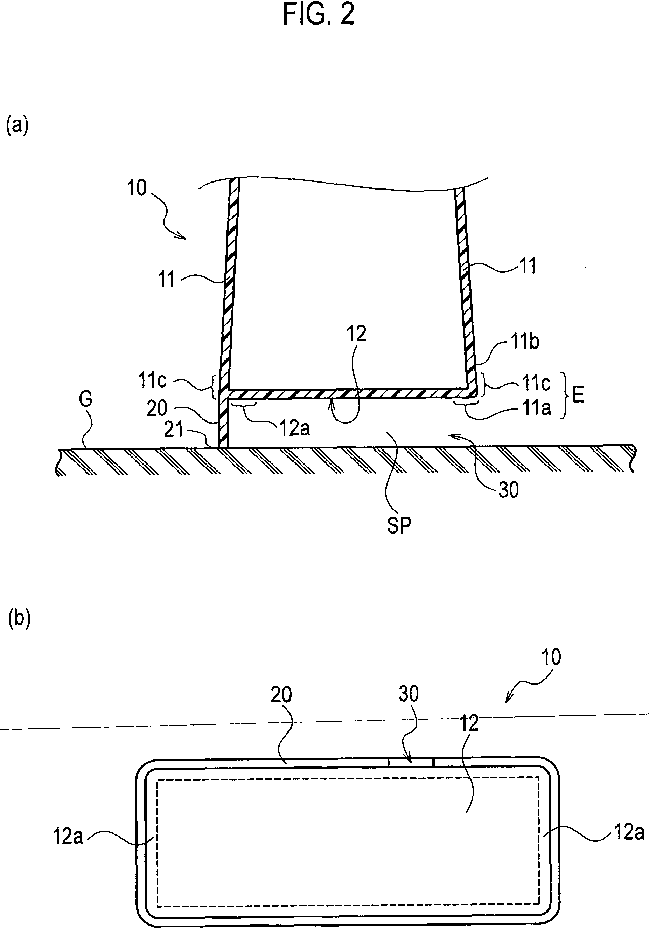

Fig. 2 (a) is a cross-sectional view in the A-A line of the package 1 and Fig. 2 (b) is a plan view as seen from the bottom surface 12 side of the package 1 (view on arrow B of Fig. 1). The skirt unit 20 extends out in an almost perpendicular direction to the bottom surface 12 from the outer edge portion E of the bottom surface 12 of the packaging bag unit 10. In the present embodiment, the skirt unit 20 extends out along the side surface 11 in an almost perpendicular direction to the bottom surface 12, and encloses the bottom surface 12. Here, the outer edge portion E may also include a lateral-side lower edge portion 11c that is a part of the side surface 11 positioned on the outer side from the outer periphery of the bottom surface 12. Furthermore, the outer edge portion E may also include a bottom-side edge portion 12a that is a part of the bottom surface 12 on the inner side from the outer periphery of the bottom surface 12. In the present embodiment, the skirt unit 20 configures an extended support unit.

In the skirt unit 20, an aperture portion 30 is formed such that the aperture portion 30 enables air flow between a space SP from the bottom surface 12 up to the placement surface, and the outside of the space SP when the package 1 is placed on the placement surface G. By including the aperture portion 30, the skirt unit 20 prevents the space formed due to enclosure by the skirt unit 20, the placement surface, and the bottom surface 12 from being closed.

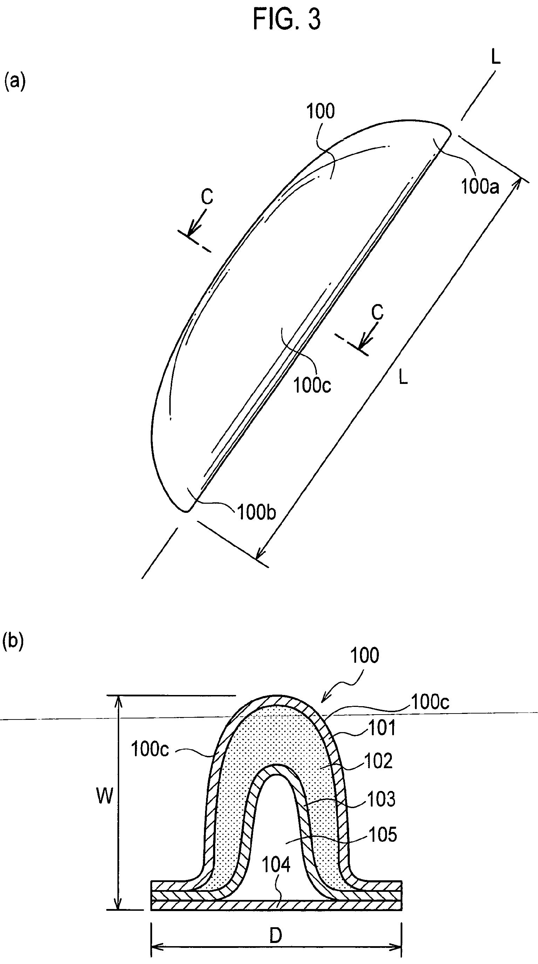

An example of the absorbent article 100 is shown in Fig. 3. Fig. 3 (a) is a perspective view for explaining an example of the absorbent article 100 contained in the package 1. Fig. 3 (b) is a cross-sectional view of the absorbent article 100 in the C-C line shown in Fig. 3 (a).

In the present embodiment, the absorbent article 100 contained in the package 1 is an interlabial pad. As shown in Fig. 3 (a), the interlabial pad (absorbent article 100) has a longitudinally elongated shape. The length L in the longitudinal direction of the absorbent article 100 is larger than the thickness D when the absorbent article 100 is folded. Furthermore, the length L in the longitudinal direction of the absorbent article 100 is also larger than the length W in the widthwise direction when the absorbent article 100 is folded.

In the present embodiment, the shape of the direction (C-C cross-section) perpendicular to the longitudinal direction of the absorbent article 100 is almost triangular, and the cross-sectional area in the C-C cross-section of the absorbent article 100 reduces towards the outer edge portion 100b of the bottom surface 12 in the longitudinal direction. That is, the outer edge portion 100b positioned on the bottom surface 12 side of the absorbent article 100 has a tapered shape that narrows as it moves towards the tip in the longitudinal direction. In the present embodiment, the outer edge portion 100a of the absorbent article 100 is also similarly tapered.

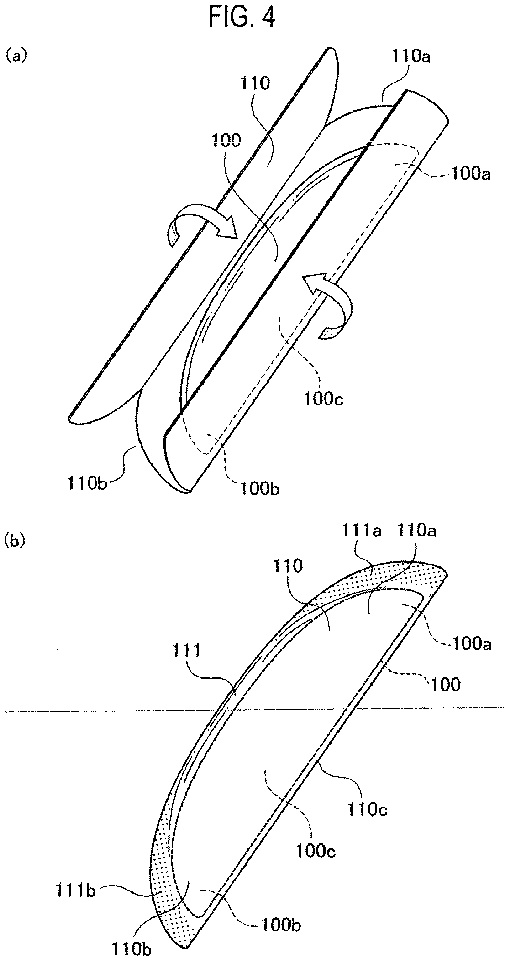

As shown in Fig. 3 (b), the absorbent article 100 has a liquid-permeable topsheet 101 that is in contact with the skin of the wearer, a backsheet 103 that is in contact with the underwear, and an absorber 102 arranged between the topsheet 101 and the backsheet 103, and these are joined with an adhesive. With the central line 1 (see Fig. 4 (a)) in the longitudinal direction as a crease, the absorbent article 100 is folded into a mountain fold having the topsheet 101 towards the outside. That is, the surface of the absorbent article 100 folded into a mountain fold forms the absorbing surface 100c. A sheet piece 104 is provided on the backsheet 103 side, which is joined with the edge portions of the absorbent article 100 in the widthwise direction. The wearer places a wearer's finger in a finger insertion opening 105 formed by the sheet piece 104 and the backsheet 103 and puts the absorbent article in the genital area.

Water-dispersible material, water-decaying material, biodegradable material, or water-soluble material that can be flushed down the toilet and processed is preferable to be used as the material configuring the absorbent article 100.

The topsheet 101 is liquid permeable. A hydrophilic, hydrolyzable material that does not irritate the skin can be used as the topsheet 101. For example, a nonwoven fabric manufactured by wet-type spun lace, for example, can be used individually or a material in which such fabrics are combined together can be used.

The material of the absorber 102 is not particularly limited as long as the material can absorb and hold liquids (bodily fluid) . A material that is bulky and does not get deformed easily, and has very few chemical irritants is desired. For example, pulp, chemical pulp, rayon, acetate rayon, natural cotton, a polymer absorber, a fibrous polymer absorber, or synthetic fibers can be used individually, or as a mixture.

The backsheet 103 has a predetermined water-resisting property in at least one part. A liquid-permeable material that allows the liquid to flow out from the backsheet 103 side to the absorber 102 side when the amount of absorption of the absorber 102 exceeds a predetermined value (for example, around 70% of the allowable absorption amount) is preferable.

The adhesive that is not shown in the drawings can be selected depending on the material of each sheet that is joined. For example, when joining together the absorber 102 and the backsheet 103 that uses hydrolyzable wet-type spun lace configured from fibers with a fiber length of 1 to 20 mm or less, a pressure-sensitive adhesive consisting mainly of synthetic rubber such as a styrene-ethylene-butadiene-styrene block copolymer (SEBS), a styrene-butadiene-styrene block copolymer (SBS), or a styrene-isoprene-styrene block copolymer (SIS), or a heat-sensitive adhesive consisting mainly of synthetic rubber such as EVA can be used as the adhesive to be applied between the sheet pieces of the backsheet and the absorber.

Next, the packaging sheet 110 that covers the absorbent article 100 is explained. Fig. 4 (a) is a schematic diagram for explaining the packaging sheet 110 that enwraps the absorbent article 100, and Fig. 4 (b) is a perspective view of the absorbent article 100 wrapped in the packaging sheet.

The absorbent article 100 is further packaged by a packaging sheet 110 that is in contact with one surface of the absorbent article 100. In the present embodiment, the packaging sheet 110 is in contact with the absorbing surface 100c of the absorbent article 100. In the present embodiment, the outer edge portion 111 of the packaging sheet 110 positioned on the outer side of the absorbent article 100 extends towards the bottom surface 12 from the outer edge portion 100b of the absorbent article 100 positioned on the bottom surface 12 side, and only an outer edge end portion 111b of the packaging sheet 110 is in contact with the bottom surface 12. That is, the absorbent article 100 is contained in the package 1 such that the surface 110c of the packaging sheet 110 that is in contact with the absorbing surface 100c of the absorbent article 100 does not come in contact with the bottom surface 12.

In the present embodiment, the outer edge portion 111 of the packaging sheet 110 that is positioned on the outer side of the absorbent article 100 is sealed by embossing or by an adhesive. In Fig. 4, of the edges that can be in contact with each other due to folding of the packaging sheet 110, the outer edge end portions 111a and 111b in the longitudinal direction of the outer edge portion 111 of the packaging sheet 110 are adhered to each other. Note that in the present embodiment, at least the outer edge end portions 111b positioned on the bottom surface side may be in contact with each other.

As described above, the package 1 includes the packaging bag unit 10 where the absorbent article 100 is placed therein, and the skirt unit 20 extending out from the outer edge portion E of the bottom surface 12 of the packaging bag unit 10 in a direction that is almost perpendicular to the bottom surface 12, and supporting the packaging bag unit to be spaced from the placement surface G on which the package 1 of the absorbent article is placed. The packaging bag unit 10 packages the absorbent article 100 such that the absorber 102 is upright with respect to the bottom surface 12.

Therefore, without the absorbing surface 100c of the absorbent article 100 being in contact with the bottom surface 12, the outer edge portion 100b of the absorbent article 100 is positioned on the bottom surface 12 side. Thus, even when moisture is generated on the inner side of the bottom surface 12 of the packaging bag unit 10 due to condensation, its absorption by the absorbing surface 100c of the absorbent article 100 can be prevented.

Furthermore, in the skirt unit 20, an aperture portion 30 is formed such that the aperture portion 30 enables air flow between the space SP from the bottom surface 12 up to the placement surface G, and the outside of the space SP when the package 1 of the absorbent article is placed on the placement surface G.

Thus, the air permeability between the space SP formed due to enclosure by the skirt unit 20, the placement surface G, and the bottom surface 12, and the outside of the space SP is improved. Thus, the temperature of the placement surface G is not transferred easily to the bottom surface 12 of the package 1, and a difference in the temperature between the space SP between the skirt unit 20 and the placement surface G, and the surroundings of the packaging bag unit 10 (outer side of the skirt unit 20) does not occur easily, which makes it difficult for the air inside the packaging bag unit 10 to build up condensation. Therefore, a decline in the absorbency power of the absorbent article 100 due to the absorption of the moisture formed as a result of condensation of the air inside the packaging bag unit 10 by the absorbent article 100 can be prevented.

Thus, according to the package 1, the absorbent article 100 can be stored without a decline in the absorbency power of the absorbent article 100.

The absorbent article 100 is further packaged by the packaging sheet 110 that is in contact with one surface of the absorbent article 100 (the absorbing surface 100c in the present embodiment). Thus, even when humid air enters inside the package 1, the absorbent article 100 can be protected from the humidity.

Furthermore, the outer edge end portion 111b of the packaging sheet 110 that is opposite the bottom surface 12 extends towards the bottom surface 12 from the outer edge portion 100b of the absorbent article positioned on the bottom surface 12 side, and only the outer edge end portion 111b of the packaging sheet 110 is in contact with the bottom surface 12. Therefore, because the outer edge portion 100b of the absorbent article 100 is spaced out from the bottom surface 12, the decline in the absorbency power of the absorbent article 100 as a result of absorption of the moisture formed due to condensation of the air inside the packaging bag unit 10 by the absorbent article 100 can be prevented.

Additionally, the absorbent article 100 is folded such that the absorbing surface 100c of the absorbent article 100 is almost perpendicular to the bottom surface 12, and the outer edge portion 100b of the absorbent article 100 that is positioned on the bottom surface 12 side has a tapered shape that narrows as it moves towards the tip. Therefore, the cross-sectional area of the absorbent article 100 opposite the bottom surface 12 is smaller at the location closer to the bottom surface 12, and the moisture formed as a result of condensation is not absorbed easily.

Furthermore, of the outer edge portions 111 of the packaging sheet 110, at least the outer edge end portions 111b of the packaging sheet 110 that are positioned on the bottom surface 12 side may be joined with each other. Therefore, even when humid air enters inside the package 1 after it has been opened, the absorbent article 100 can be protected from the humidity.

Furthermore, by providing a re-sealable chuck or seal at a predetermined position on the side surface 11 of the packaging bag unit 10 of the package 1, the package 1 can be sealed in cases other than when the user takes out the absorbent article 100, and thus the entry of humidity from outside can be prevented.

### (Modifications of the skirt unit)

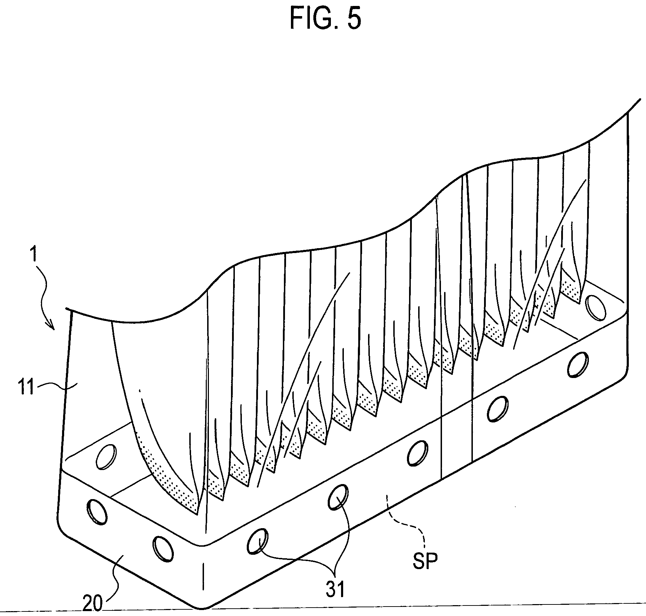

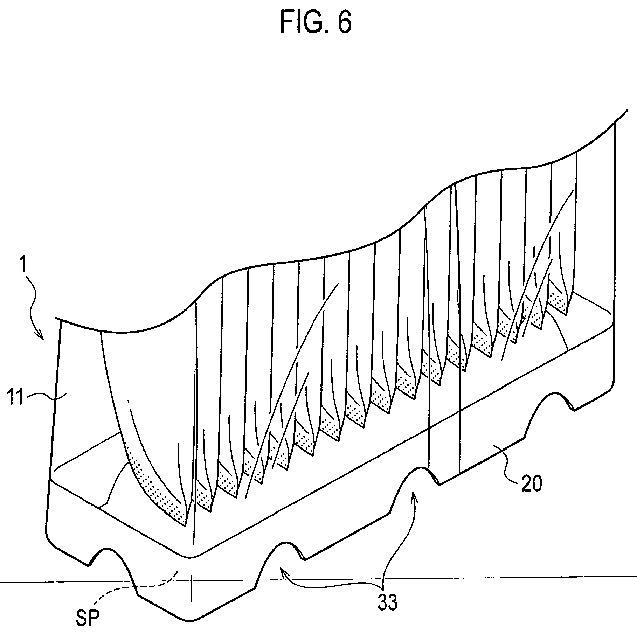

Fig. 5 and Fig. 6 are enlarged views for explaining modifications of the skirt unit. In the modification shown in Fig. 5, the aperture portion is a circular through-hole formed in the skirt unit 20. A plurality of through-holes 31 may be formed in the skirt unit 20. In the modification shown in Fig. 6, the aperture portion is a notch formed from the end portion 21 of the skirt unit 20 towards the bottom surface 12. A notch 33 is almost semicircular in shape.

As shown in Fig. 5 and Fig. 6, when the through-holes 31 or the notches 33 are formed in the skirt unit 20 of the package 1, the air permeability between the space SP formed due to enclosure by the skirt unit 20, the placement surface G, and the bottom surface 12, and the outside of the space SP can be improved.

Thus, in the package 1, a difference in the temperature between the space SP between the skirt unit 20 and the placement surface G, and the surroundings of the packaging bag unit 10 (that is, the outer side of the skirt unit 20) does not occur easily, which makes it difficult for the air inside the packaging bag unit 10 to build up condensation. Therefore, a decline in the absorbency power of the absorbent article 100 due to the absorption of the moisture formed as a result of condensation of the air inside the packaging bag unit 10 by the absorbent article 100 can be prevented more effectively. Thus, according to the package 1, the absorbent article 100 can be stored without a decline in the absorbency power of the absorbent article 100.

### (Method of manufacturing package 1)

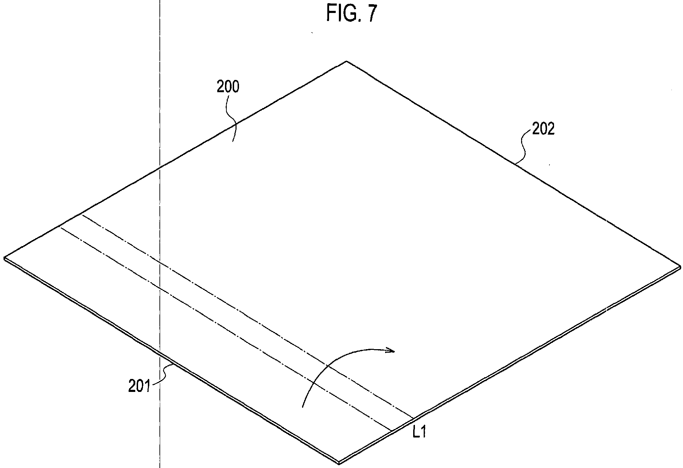

Next, a method of manufacturing the package 1 is explained. Fig. 7 to Fig. 11 are assembly drawings of the package 1. The method of manufacturing the package 1 in which the side surface 11 of the packaging bag unit 10, the bottom surface 12, and the skirt unit 20 form one unit is shown by using Fig. 7 to Fig. 11.

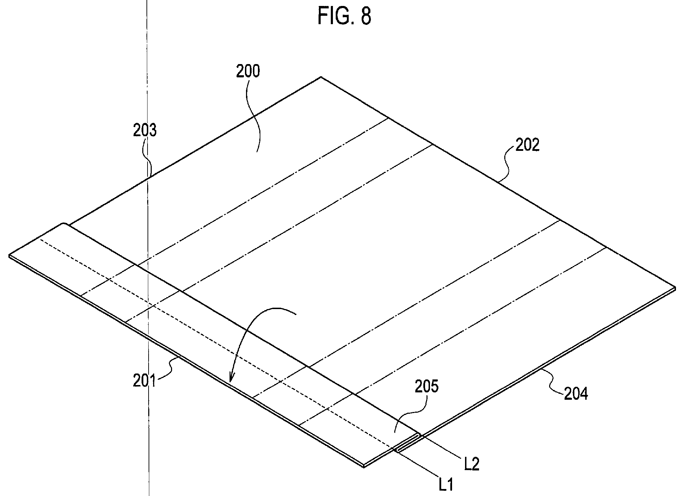

The package 1 is formed from a sheet-like material 200. The sheet-like material 200 has an end portion 201 and an end portion 202. As shown in Fig. 7 and Fig. 8, with a virtual line L1 positioned between one end portion 201 and the other end portion 202 of the continuous sheet-like material 200 that forms the packaging bag unit 10 as the first crease, one end portion 201 is folded back towards the other end portion 202.

With a virtual line L2 positioned between the first crease (virtual line L1) and the other end portion 202 in the folded-back sheet-like material 200 as the second crease, one end portion 201 is folded back towards the first crease (virtual line L1). Thus, a ruffled portion 205 corresponding to the skirt unit 20 is formed. The virtual lines L1 and L2 run along the widthwise direction of the sheet-like material 200.

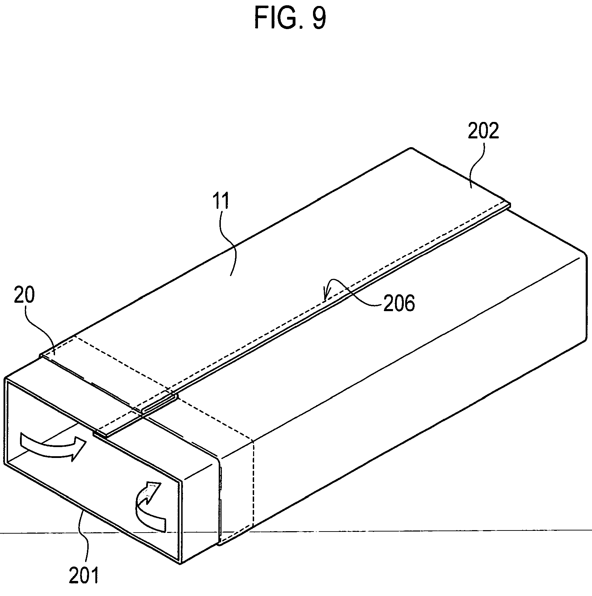

As shown in Fig. 9, side edges 203 and 204 of the sheet-like material 200 on which the ruffled portion 205 (skirt unit 20) is formed are overlapped with each other to form a cylindrical shape, and a rear selvage portion 206 is formed by fusing together the side edges 203 and 204.

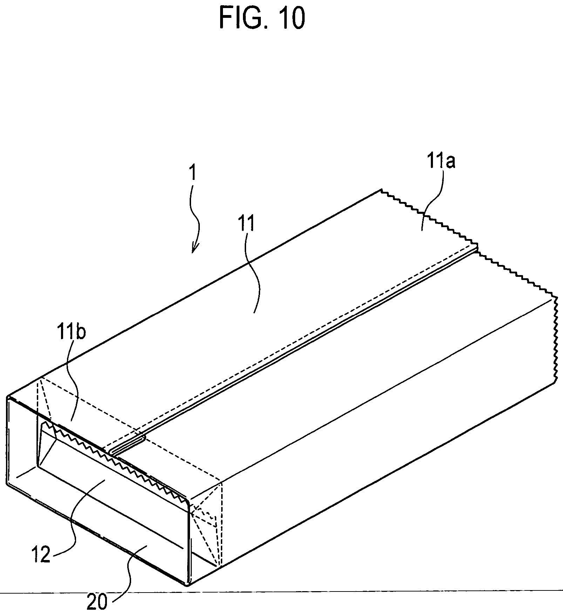

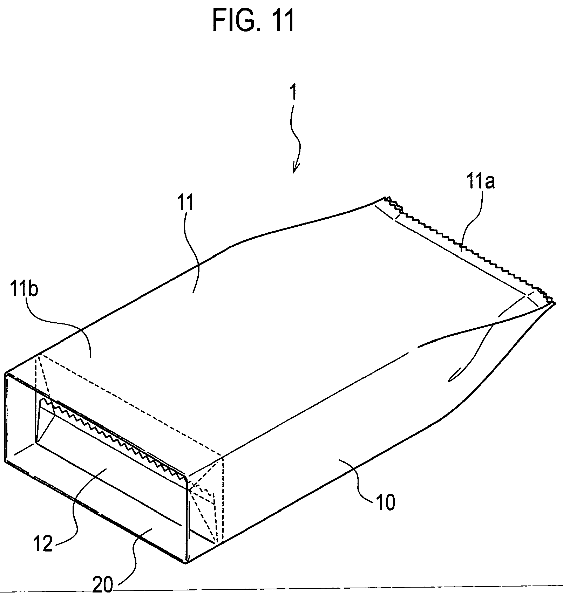

Next, as shown in Fig. 10 and Fig. 11, the top and bottom aperture portions are sealed. The bottom surface 12 is formed by folding and joining one end portion 201. The bottom surface 12 is folded towards the inside of the skirt unit 20. To securely fit the bottom surface 12 inside the skirt unit 20, the end portions of the bottom surface 12 can be fixed with an adhesive tape or HMA. As described above, the package 1 with an integrated skirt unit 20 can be manufactured.

### (Modification of the manufacturing method)

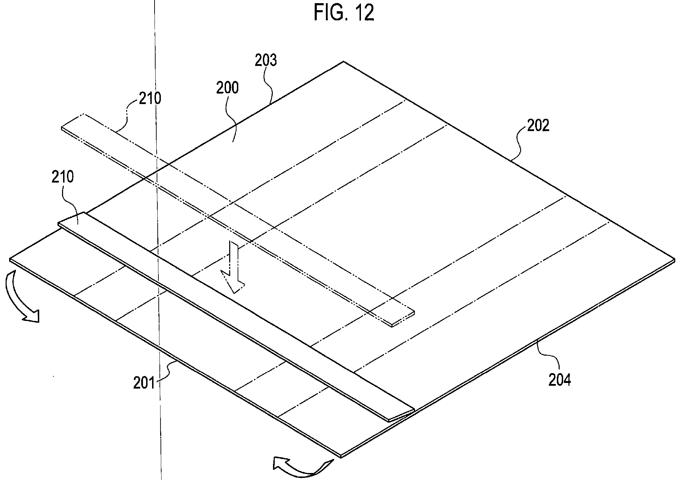

According to the method of manufacturing the package 1, the skirt unit 20 prepared as a separate member may be pasted on to the side surface 11 of the packaging bag unit 10. According to the manufacturing method, rather than forming the ruffled portion 205 (skirt unit 20) by a two-stage folding step of the sheet-like material 200 as shown in Fig. 8, a band-shaped member 210 corresponding to the skirt unit 20 is joined at a predetermined position of the continuous sheet-like material 200 that forms the packaging bag unit 10, as shown in Fig. 12.

Following this, the side edges 203 and 204 of the sheet-like material 200 on which the band-shaped member 210 corresponding to the skirt unit 20 is formed are joined together to form a cylindrical shape. After forming a cylindrical shape, the package 1 including the skirt unit 20 can be manufactured according to the aforementioned step.

### 2. Second embodiment

### (Structure of package 2)

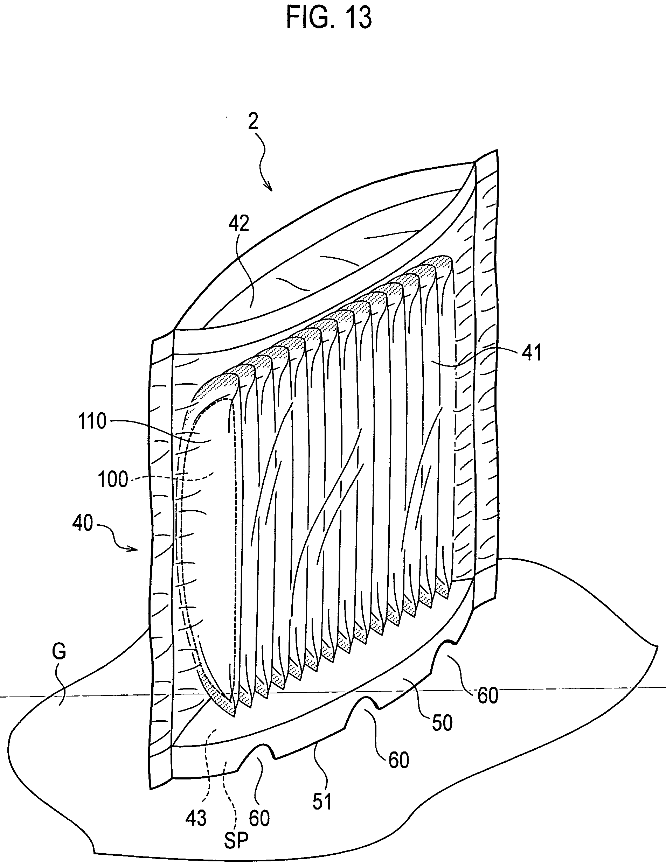

Fig. 13 is a perspective view for explaining a package 2 shown as a second embodiment of the present invention. The package 2 is formed from a sheet-like material including a resin sheet, and packages the absorbent article. The package 2 includes a packaging bag unit 40 configured to package the absorbent article 100 and a skirt unit 50 provided on the surface of the packaging bag unit 40.

The packaging bag unit 40 includes a side surface 41, an upper bottom surface 42, and a lower bottom surface 43. The side surface 41 may be provided with a mechanism (such as a chuck or seal) that enables re-sealing after the seal has been opened once.

The skirt unit 50 extends along the side surface 41 and encloses the lower bottom surface 43. An end portion 51 of the skirt unit 50 is in contact with the placement surface on which the package 2 is placed. That is, the package 2 is in an upright state with the lower bottom surface 43 at the bottom by being supported by the skirt unit 50. Aperture portions 60 are formed in the skirt unit 50.

Because the lower bottom surface 43 of the package 2 does not come in direct contact with the placement surface as the package 2 is supported by the skirt unit 50, the temperature of the placement surface is not transferred easily to the lower bottom surface 43 even when the package 2 is placed at a location with high humidity and large temperature difference such as a toilet, a washroom, or a dressing room. Furthermore, when the aperture portions 60 are formed in the skirt unit 50 of the package 2, the air permeability between the space SP formed due to enclosure by the skirt unit 50, the placement surface G, and the lower bottom surface 43, and the outside of the space SP is improved.

Thus, in the package 2, a difference in the temperature between the space SP formed by the lower bottom surface 43, the skirt unit 50, and the placement surface G, and the surroundings of the packaging bag unit 40 (that is, the outer side of the skirt unit 50) does not occur easily, which makes it difficult for the air inside the packaging bag unit 10 to build up condensation. Therefore, a decline in the absorbency power of the absorbent article 100 due to the absorption of the moisture formed as a result of condensation of the air inside the packaging bag unit 40 by the absorbent article 100 can be prevented more effectively.

Thus, according to the package 2, the absorbent article 100 can be stored without a decline in the absorbency power of the absorbent article 100.

### (Method of manufacturing the package 2)

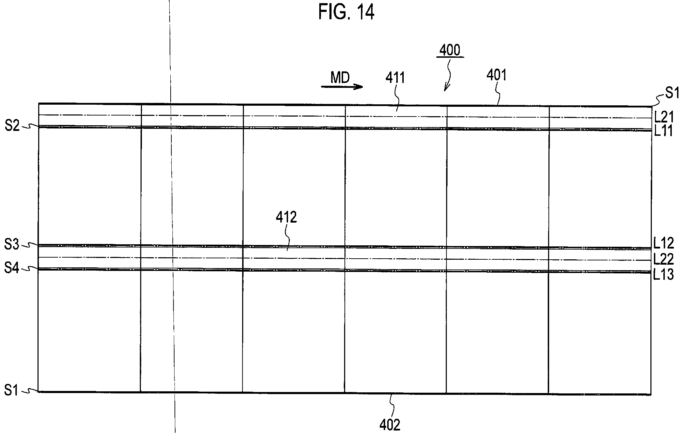

Next, a method of manufacturing the package 2 is explained. Fig. 14 to Fig. 16 are assembly drawings of the package 2.

The package 2 is formed from a continuous body of a sheet-like material 400. The continuous body of the sheet-like material 400 has an end portion 401 and an end portion 402 in the longitudinal direction. As shown in Fig. 14, a virtual line L11 is specified at one end portion 401 side of the sheet-like material 400. Furthermore, a virtual line L21 is specified between the virtual line L11 and the end portion 401. Furthermore, virtual lines L12 and L13 are specified between the end portion 401 and the end portion 402. Additionally, a virtual line L22 is specified between the virtual lines L12 and L13. A region 411 formed by the virtual line L11 and the end portion 401 configures the upper bottom surface 42 (see Fig. 13). A region 412 formed by the virtual lines L12 and L13 configures the lower bottom surface 43 (see Fig. 14).

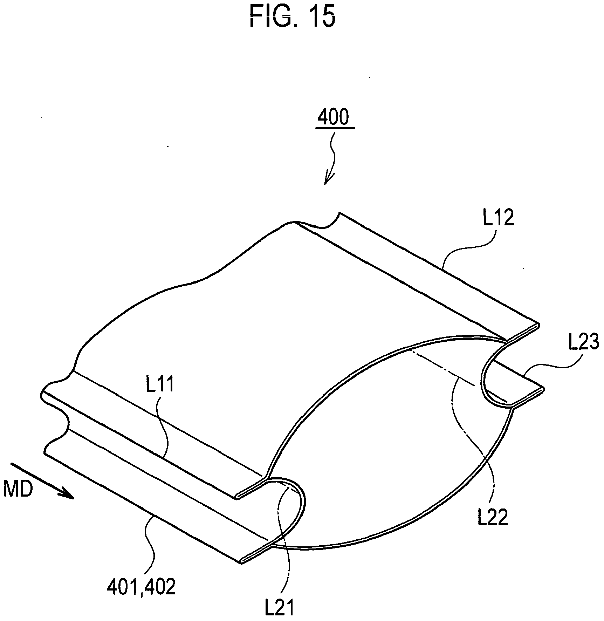

The end portion 401 and the end portion 402 are joined together while the continuous body of the sheet-like material 400 is conveyed along a conveyance direction MD shown in Fig. 14. Thus, the cylindrical continuous body shown in Fig. 15 is formed. At the positions of the virtual lines L11, L12, and L13 of the cylindrical continuous body shown in Fig. 15, the continuous body is folded into a mountain fold protruding towards the outside of the cylinder, and at the positions of the virtual lines L21 and L22, the continuous body is folded into a valley fold.

A region S1, the region S1 including the virtual line L11, a region S3 including the virtual line L12, and a region S4 including the virtual line L13, which are shown in Fig. 14, are portions that are fused together by thermal fusion bonding. The region S1 serves as a joint unit between the end portion 401 and the end portion 402. By storing the absorbent article 100 in the cylindrical continuous body shown in Fig. 15, and then fusing together joint regions S7 through thermal fusion bonding, for example, a continuous body of the package 2 in which the absorbent article 100 has been packaged can be manufactured.

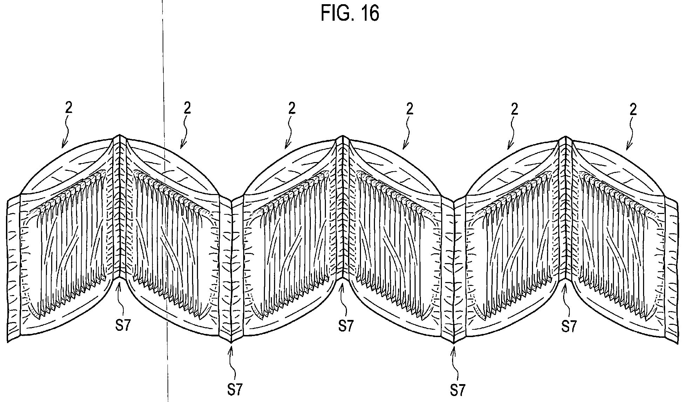

Fig. 16 is a perspective view of a continuous body of the package 2. As shown in Fig. 16, a plurality of the packages 2 can also be sold in a continuous state. Furthermore, the continuous body of the packages 2 can also be cut off at the joint region S7 and sold individually.

### 3. Other embodiments

As described above, the present invention is disclosed through the above embodiments. However, it should not be interpreted that the statements and drawings constituting a part of the present disclosure limit the present invention. From this disclosure, various alternate embodiments, examples, and operation technology will become apparent to one skilled in the art.

For example, in the embodiments of the present invention, a case in which the absorbent article is an interlabial pad was explained, however, the absorbent article is not limited thereto. For example, the absorbent article maybe a sanitary napkin, a diaper, an inner liner, or an incontinence pad.

In the embodiment, the package 1 in which an absorber 102 of the absorbent article 100 is packaged in an upright state with respect to the bottom surface 12 was explained. However, for example, the package includes those in which an absorbent article where the absorbing surface of the absorber is not flat, such as a tampon, is packaged such that the longitudinal direction of the absorbent article and the up-and-down direction of the package are almost matching.

Furthermore, in the embodiments, it is explained that the aperture portion formed in the skirt unit 20 or 50 is either a through-hole or a notch. However, the aperture portion can also be slit shaped with a narrow width as compared to the length. A plurality of the through-holes 31 are formed in the skirt unit 20 shown in Fig. 5. However, the single through-hole 31 can also be formed in the skirt unit 20. Similarly, a plurality of the notches 33 are formed in the skirt unit 20 shown in Fig. 6. However, the single notch 33 can also be formed in the skirt unit 20.

The shape of the aperture portion 31 formed in the skirt unit 20 of the package 1 is not limited to a circle as shown in Fig. 5. For example, the aperture portion may be an ellipse, a triangle, a quadrangle, a trapezoid, heart shaped, star shaped, flower shaped, or the silhouette of a character. Furthermore, the shape of the notch 33 formed in the skirt unit 20 of the package 1 is not limited to a semi-circle as shown in Fig. 6. For example, the notch may be a curve, a rectangular shape, a wavy line, or zigzag, and not necessarily a semi-circle.

Furthermore, the size and interval of the aperture portion 31 and the notch 33 is also not limited to that shown in Fig. 6 or Fig. 7. The aperture portion 31 and the notch 33 can be formed appropriately as long as the skirt units 20 and 50 do not lose the strength necessary for supporting the packaging bag unit 10 in which the absorbent article 100 is contained.

Furthermore, in the embodiments, the skirt units 20 and 50 were illustrated as an example of the extended support unit. However, leg portions provided at the four corners of the bottom surface 12 of the packaging bag unit 10, and supporting the package 1 at the four points of the four corners are also included as the extended support units, for example.

In the packages 1 and 2 of the aforementioned embodiments, an indicator (thermochromism) whose color changes depending on the temperature can be arranged at a visible location in the skirt unit 20 or the skirt unit 50. Furthermore, a pigment having an indicator function can also be applied directly at a visible location. The user can know the temperature of the package by looking at the indicator. Therefore, it is possible to take precaution so as not to suddenly move the package to a warm room if it had been kept on a cool placement surface.

In the package 1 according to the present embodiment, a desiccant such as silica gel can also be pasted with an adhesive member on to the bottom surface at the contain side of the packaging bag unit 10. This will enable a reduction in the humidity inside the package.

Furthermore, a deadweight can also be attached on the inner side of the package 1 according to the present embodiment, or on the outer surface of the bottom surface 12. In such a case, the package can be stabilized with the load of the deadweight. However, the deadweight must be such that the bottom surface of the package to which the deadweight is attached does not come in contact with the placement surface due to the self-load of the deadweight. When attaching the deadweight to the outer surface of the bottom surface 12, the deadweight must have a size that can be fitted in the space formed by the skirt unit 20, the placement surface, and the bottom surface 12, and the bottom surface 12 of the package must not come in contact with the placement surface due to the self-load of the deadweight.

Besides attaching the deadweight to the skirt unit 20, silicon coating can also be performed. Thus, the weight of the skirt unit 20 increases, and the stability can be increased. Furthermore, the anti-slip effect can also be increased by the silicon coating, and therefore, the turning over of the package 1 can be prevented.

In the method of manufacturing the package 1 shown as the embodiment, the interval between the virtual lines L1 and L2 is not limited to the example shown in Fig. 8. By increasing the interval between the virtual lines L1 and L2, the length of the skirt unit 20 from the placement surface G can be adjusted. That is, the height of the bottom surface 12 from the placement surface G can be changed.

In the method of manufacturing the package 1 shown in Fig. 7 to Fig. 11, the number of times the sheet-like material 200 is folded back to form the skirt unit 20 is not limited. If the folding frequency is increased, the thickness of the skirt unit 20 increases, and thus the strength of the skirt unit 20 can be increased. The folding frequency can also be changed depending on the weight of the product of the absorbent article 100 contained in the package 1.

In the modification of the manufacturing method shown in Fig. 12, the width and attachment location of the band-shaped member 210 with respect to the side edges 203 and 204 of the sheet-like material 200 is not limited to the example shown in Fig. 12. For example, by increasing the width of the band-shaped member 210 with respect to the side edges 203 and 204, and by attaching the band-shaped member at a location closer to the center of the sheet-like material 200 as compared to the attachment location shown in Fig. 12, the stability in the upright state can be improved. The width and attachment location can be changed depending on the size and weight of the product of the absorbent article 100 contained in the package 1.

As described above, needless to say, the present invention includes various embodiments and the like not described herein. Therefore, the scope of the present invention is to be defined only by the matters specifying the invention according to the claims in view of the above description.

### [Example]

Next, an example is explained. An example of implementing the package 1 of the aforementioned first embodiment is explained. The length of the absorbent article contained in the package in the longitudinal direction is 135 mm, the width is 35 mm, and the thickness is 7 mm. The design is such that the absorbent article having the aforementioned size can be contained while bringing the end portions in the longitudinal direction of the absorbent article and the bottom surface of the packaging bag unit into contact with each other.

In the package, the length of the skirt unit from the placement surface up to the bottom surface is 13 mm. The packaging becomes unstable when the length of the skirt unit becomes too long with respect to the length of the absorbent article. Preferably, the length of the skirt unit is 5 mm or more. The skirt unit is formed by folding back the sheet-like material two times according to the step shown in Fig. 8. The inner surface of the folded back portion is adhered. An orthophenyl phenol and a polypropylene film are superposed and used as the sheet-like material configuring the packaging bag unit.

### [Applicability]

According to the present invention, it is possible to provide a package of an absorbent article that can effectively prevent a decline in the absorbency power of the absorbent article due to the absorption of the moisture formed as a result of condensation of the air inside the packaging bag owing to the difference in the temperature between the space between the packaging bag and the placement surface, and the surroundings of the packaging bag.

## Claims

1. A package (1) of an absorbent article (100) in which the absorbent article having an absorber (102) that absorbs moisture is packaged in a sealed state, comprising:
a packaging bag unit (10) where the absorbent article is placed therein;
wherein the packaging bag unit packages the absorbent article such that the absorber is in an upright state with respect to the bottom surface **characterised in that** said packaging bag unit comprises an extended support (30) unit extending out from an outer edge portion (E) of a bottom surface (12) of the packaging bag unit in a direction that is almost perpendicular to the bottom surface (12), and configured to support the packaging bag unit to be spaced from a placement surface (G) on which the package of the absorbent article is placed.

2. The package of the absorbent article according to claim 1, wherein the absorbent article is further packaged by a packaging sheet (110) that covers one surface of the absorbent article, an outer edge portion (111) of the packaging sheet opposite the bottom surface extends towards the bottom surface from the outer edge portion (100b) of the absorbent article positioned on the bottom surface side, and only the outer edge portion of the packaging sheet is in contact with the bottom surface.

3. The package of the absorbent article according to claim 1 or 2, wherein the absorbent article is folded such that the absorber is in an upright state with respect to the bottom surface, and the outer edge portion of the absorbent article positioned on the bottom surface side has a tapered shape that narrows towards the tip.

4. The package of the absorbent article according to claim 2 or 3, wherein the absorbent article packaged by the packaging sheet is folded such that the absorber is in an upright state with respect to the bottom surface, and of the outer edge portions of the packaging sheet that can be brought in contact with each other by folding the packaging sheet, at least one outer end portion of the packaging sheet positioned on the bottom surface side is joined to another outer end portion.

5. The package of the absorbent article according to any one of claims 1 through 4, wherein an aperture portion (30) is formed in the extended support unit such that the aperture portion enables air flow between a space from the bottom surface up to the placement surface, and the outside of the space when the package of the absorbent article is placed on the placement surface.

6. The package of the absorbent article according to any one of claims 1 through 5, wherein the packaging bag unit packages the absorbent article such that an absorbing surface of the absorber is almost perpendicular with respect to the bottom surface.

## Patentansprüche

1. Eine Verpackung (1) für einen absorptionsfähigen Artikel (100), wobei der absorptionsfähige Artikel, der einen Feuchtigkeit absorbierenden Absorber (102) aufweist, in einem abgedichteten Zustand verpackt ist, beinhaltend:
eine Verpackungsbeuteleinheit (10), wobei der absorptionsfähige Artikel darin platziert ist;
wobei die Verpackungsbeuteleinheit den absorptionsfähigen Artikel derart verpackt, dass sich der Absorber in Bezug auf eine untere Oberfläche in einem aufrechten Zustand befindet, **dadurch gekennzeichnet, dass** die Verpackungsbeuteleinheit eine verlängerte Stützeinheit (30) beinhaltet, die sich von einem äußeren Randabschnitt (E) der unteren Oberfläche (12) der Verpackungsbeuteleinheit her in eine Richtung erstreckt, die nahezu senkrecht zu der unteren Oberfläche (12) verläuft, und zum Stützen der Verpackungsbeuteleinheit, die von einer Platzierungsoberfläche (G), auf welcher die Verpackung des absorptionsfähigen Artikels platziert wird, mit Abstand angeordnet werden soll, konfiguriert ist.

2. Verpackung für einen absorptionsfähigen Artikel gemäß Anspruch 1, wobei der absorptionsfähige Artikel weiterhin mit einer Verpackungsfolie (110) verpackt wird, die eine Oberfläche des absorptionsfähigen Artikels abdeckt, wobei sich ein äußerer Randabschnitt (111) der Verpackungsfolie gegenüber der unteren Oberfläche zur unteren Oberfläche von dem äußeren Randabschnitt (100b) des absorptionsfähigen Artikels, positioniert auf der Seite der unteren Oberfläche, erstreckt, und wobei lediglich der äußere Randabschnitt der Verpackungsfolie mit der unteren Oberfläche in Kontakt steht.

3. Verpackung für einen absorptionsfähigen Artikel gemäß Anspruch 1 oder 2, wobei der absorptionsfähige Artikel derart gefaltet ist, dass sich der Absorber in Bezug auf die untere Oberfläche in einem aufrechten Zustand befindet, und der auf der Seite der unteren Oberfläche positionierte äußere Randabschnitt des absorptionsfähigen Artikels eine sich verjüngende Form aufweist, die sich zur Spitze hin verschmälert.

4. Verpackung für einen absorptionsfähigen Artikel gemäß Anspruch 2 oder 3, wobei der mit der Verpackungsfolie verpackte, absorptionsfähige Artikel derart gefaltet ist, dass sich der Absorber in Bezug auf die untere Oberfläche in einem aufrechten Zustand befindet, und wobei von den äußeren Randabschnitten der Verpackungsfolie, die durch Falten der Verpackungsfolie miteinander in Kontakt gebracht werden können, mindestens ein auf der Seite der unteren Oberfläche positionierter äußerer Endabschnitt der Verpackungsfolie an einen anderen äußeren Endabschnitt gefügt ist.

5. Verpackung für einen absorptionsfähigen Artikel gemäß einem der Ansprüche 1 bis 4, wobei ein Öffnungsabschnitt (30) in der verlängerten Stützeinheit ausgebildet ist, sodass der Öffnungsabschnitt das Strömen von Luft zwischen einem Raum von der unteren Oberfläche bis nach oben zur Platzierungsoberfläche und der Außenseite des Raums ermöglicht, wenn die Verpackung des absorptionsfähigen Artikels auf der Platzierungsoberfläche platziert wird.

6. Verpackung für einen absorptionsfähigen Artikel gemäß einem der Ansprüche 1 bis 5, wobei die Verpackungsbeuteleinheit den absorptionsfähigen Artikel derart verpackt, dass eine absorptionsfähige Oberfläche des Absorbers mit Bezug auf die untere Oberfläche nahezu senkrecht verläuft.

## Revendications

1. Un emballage (1) d'un article absorbant (100) dans lequel l'article absorbant ayant un absorbeur (102) qui absorbe l'humidité est emballé dans un état hermétique, comprenant :
une unité de sac d'emballage (10), dans laquelle l'article absorbant est placé ;
dans lequel l'unité de sac d'emballage emballe l'article absorbant de telle sorte que l'absorbeur est dans un état droit par rapport à une surface de dessous, **caractérisé en ce que** ladite unité de sac d'emballage comprend une unité de support étendue (30) s'étendant vers l'extérieur depuis une portion de bord externe (E) de la surface de dessous (12) de l'unité de sac d'emballage dans une direction qui est presque perpendiculaire à la surface de dessous (12), et configuré pour supporter l'unité de sac d'emballage pour qu'elle soit espacée d'une surface de placement (G) sur laquelle l'emballage de l'article absorbant est placé.

2. L'emballage de l'article absorbant selon la revendication 1, dans lequel l'article absorbant est en outre emballé par un feuillet d'emballage (110) qui recouvre une surface de l'article absorbant, une portion de bord externe (111) du feuillet d'emballage opposée à la surface de dessous s'étend vers la surface de dessous depuis la portion de bord externe (100b) de l'article absorbant positionné sur le côté surface de dessous, et seule la portion de bord externe du feuillet d'emballage est en contact avec la surface de dessous.

3. L'emballage de l'article absorbant selon la revendication 1 ou la revendication 2, dans lequel l'article absorbant est plié de telle sorte que l'absorbeur soit dans un état droit par rapport à la surface de dessous, et la portion de bord externe de l'article absorbant positionnée sur le côté surface de dessous a une forme effilée qui rétrécit vers le bout.

4. L'emballage de l'article absorbant selon la revendication 2 ou la revendication 3, dans lequel l'article absorbant emballé par le feuillet d'emballage est plié de telle sorte que l'absorbeur soit dans un état droit par rapport à la surface de dessous, et parmi les portions de bord externes du feuillet d'emballage qui peuvent être amenées en contact les unes avec les autres en pliant le feuillet d'emballage, au moins une portion d'extrémité externe du feuillet d'emballage positionnée sur le côté surface de dessous est jointe à une autre portion d'extrémité externe.

5. L'emballage de l'article absorbant selon n'importe laquelle des revendications 1 à 4, dans lequel une portion d'ouverture (30) est formée dans l'unité de support étendue de telle sorte que la portion d'ouverture permette un écoulement d'air entre un espace de la surface de dessous jusqu'à la surface de placement, et l'extérieur de l'espace lorsque l'emballage de l'article absorbant est placé sur la surface de placement.

6. L'emballage de l'article absorbant selon n'importe laquelle des revendications 1 à 5, dans lequel l'unité de sac d'emballage emballe l'article absorbant de telle sorte qu'une surface absorbante de l'absorbeur soit presque perpendiculaire par rapport à la surface de dessous.
